(19) Europäisches Patentamt
European Patent Office
Office européen des brevets

(11) EP 4 755 406 A1

(12) EUROPEAN PATENT APPLICATION
published in accordance with Art. 153(4) EPC

(43) Date of publication:
**10.06.2026 Bulletin 2026/24**

(21) Application number: **24849032.8**

(22) Date of filing: **25.07.2024**

(51) International Patent Classification (IPC):
***A61L 12/14*** (2006.01) ***A61K 31/785*** (2006.01)
***A61K 47/02*** (2006.01) ***A61K 47/18*** (2017.01)
***A61L 12/08*** (2006.01) ***A61L 12/10*** (2006.01)
***A61L 12/12*** (2006.01) ***A61P 33/04*** (2006.01)
***A61L 101/16*** (2006.01) ***A61L 101/34*** (2006.01)
***A61L 101/46*** (2006.01)

(52) Cooperative Patent Classification (CPC):
**A61K 31/785; A61K 47/02; A61K 47/18; A61L 12/08; A61L 12/10; A61L 12/12; A61L 12/14; A61P 33/04;** A61L 2101/00

(86) International application number:
**PCT/JP2024/026550**

(87) International publication number:
**WO 2025/028377 (06.02.2025 Gazette 2025/06)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **28.07.2023 JP 2023123521**

(71) Applicant: **Seed Co., Ltd.**
**Tokyo 113-8402 (JP)**

(72) Inventors:
- **OTAKE, Hideyuki**
  **Tokyo 113-8402 (JP)**
- **MURAKAMI, Ayana**
  **Tokyo 113-8402 (JP)**
- **WADA, Miyu**
  **Tokyo 113-8402 (JP)**

(74) Representative: **Schulz Junghans Patentanwälte PartGmbB**
**Großbeerenstraße 71**
**10963 Berlin (DE)**

# (54) ANTI-ACANTHAMOEBA LIQUID FORMULATION

(57) The objective of the present invention is to provide an anti-Acanthamoeba liquid formulation having anti-Acanthamoeba activity while being safe to be less effective on ocular tissue and versatile enough to be applicable to a wide range of contact lenses. The objective can be achieved by an anti-Acanthamoeba liquid formulation, comprising sodium dihydrogen phosphate, a basic isotonic agent, and poly(diallyldimethylammonium chloride) with an average molecular weight of 10,000 to 1,100,000; and the like.

EP 4 755 406 A1

**Description**

Technical Field

The present invention relates to a liquid formulation having a disinfecting effect against Acanthamoeba.

Background Art

Acanthamoeba corneal infection is a corneal infection caused by the invasion of the cornea by microorganisms of the genus *Acanthamoeba* (hereafter simply referred to as Acanthamoeba). Patients suffering from Acanthamoeba corneal infection experience severe ocular pain, and in severe cases, the cornea may be punctured, leading to blindness. The number of Acanthamoeba corneal infection cases has been increasing in recent years.

Acanthamoeba is a protozoan that is widespread in soil and freshwater, and is also found in tap water. Normally, Acanthamoeba is unlikely to cause the infection to the cornea in a healthy state. However, when there is a wound on the cornea, Acanthamoeba invades from the wound area and adhere thereto, resulting in the infection. In addition, contact lens wearers can develop Acanthamoeba corneal infection by wearing contact lenses contaminated with Acanthamoeba from tap water and other sources.

Typically, Acanthamoeba corneal infection treatment is carried out by scraping off the cornea and administration of an antifungal agent. However, Acanthamoeba corneal infection is a refractory infectious disease because the infection is difficult to be diagnosed at an early stage, and the burden to the patients is heavier due to problems such as easily recurrence of inflammation thereby prolonged treatment time and scarring of the cornea. The contact lens wearers can prevent or treat the corneal infection by using a liquid formulation containing an agent with anti-Acanthamoeba activity capable of preventing contamination of Acanthamoeba.

As such a liquid formulation, the following formulations are known: the anti-Acanthamoeba disinfectant and preservative agent for contact lenses, containing an effective amount of polylysine (e.g., see Patent Document 1); the anti-Acanthamoeba composition containing as an active ingredient lactoferrin that is a glycoprotein, or lactoferricin that is a peptide of its enzymatic degradation product (e.g., see Patent Document 2); the antiprotozoan composition containing as active ingredients the candin-based drug caspofungin and a biguanide-based compound (e.g., see Patent Document 3).

On the other hand, in recent years, Multi-Purpose Solution (MPS) can clean, rinse, disinfect and store contact lenses by one solution, and thus is easy to handle and is widely used. Organic nitrogen-based disinfectants are commonly used as disinfectant ingredients in MPS. The known disinfectants applicable to MPS include the preventive and therapeutic formulation for Acanthamoeba corneal infection, containing two active ingredients, the organic nitrogen-based disinfectant and diallyldialkylammonium-containing polymer (e.g., see Patent Document 4). Also known is the method of combining alexidine with polyhydric alcohol and boric acid to enhance antiseptic activity against Acanthamoeba (e.g., see Patent Document 5).

Citation List

Patent Documents

Patent Document 1: JP 2002-143277 A
Patent Document 2: JP 2011-246458 A
Patent Document 3: JP 2013-234176 A
Patent Document 4: JP 2014-218461 A
Patent Document 5: JP 2018-035151 A

Summary of the Invention

Problems to be Solved by the Invention

However, the formulations, compositions and methods described in Patent Documents 1 to 5 have problems, respectively.

The anti-Acanthamoeba disinfectant and preservative agent described in Patent Document 1 has problems to lack versatility since the active ingredient polylysine is not applicable to soft contact lenses of the Group IV (ionic, high water content).

The anti-Acanthamoeba composition described in Patent Document 2 has problems to be less safe because the

active ingredient glycoprotein or its degradation product is deposited in ocular tissue, resulting in visual and ocular disorders.

The antiprotozoan composition described in Patent Document 3 has problems to be less safe because the active ingredient caspofungin exhibits the antifungal activity by inhibiting the activity of 1,3-beta-glucan synthase, a major component of cell wall, but causes side effects such as anaphylaxis, liver dysfunction and ocular pruritus.

The preventive and therapeutic formulation for Acanthamoeba corneal infection described in Patent Document 4 contains as the active ingredient a high concentration of diallyldialkylammonium-containing polymer, and has problems to possibly cause side effects such as anaphylaxis, liver dysfunction and ocular pruritus due to the polymer.

The method described in Patent Document 5 is characterized by combining alexidine with polyhydric alcohol and boric acid. Among them, boric acid has been widely used in ophthalmic solutions because of its antiseptic effect. However, in recent years, it has been reported that boric acid can damage the cornea, resulting in ocular disorder and the like. In particular, the use of boric acid in ophthalmic liquid formulations has been being regulated.

Therefore, although in the formulations, compositions and methods described in Patent Documents 1 to 5, the active ingredients exhibit a disinfecting effect against Acanthamoeba, they have problems in terms of safety for ocular tissue and versatility in application to contact lenses.

In view of the above circumstances, it is an objective of the present invention to provide an anti-Acanthamoeba liquid formulation having anti-Acanthamoeba activity while being safe to be less effective on ocular tissue and versatile enough to be applicable to a wide range of contact lenses.

## Means of Solving the Problems

In order to solve the above problems, the present inventors have conducted intensive research on a liquid formulation having a disinfecting effect against Acanthamoeba while exhibiting little adverse effect on ocular tissue. In the course of the research, it was found that the combination of sodium dihydrogen phosphate and a basic isotonic agent, which have been proven to be safe for ocular tissue, exhibited a disinfecting effect against fungi but had no effect against Acanthamoeba.

As a result of further trial and error, the present inventors have finally arrived at the use of poly(diallyldimethylammonium chloride), which has been also proven to be safe for ocular tissue, and have further found that the combination of the predetermined poly(diallyldimethylammonium chloride) with sodium dihydrogen phosphate and a basic isotonic agent exhibits an excellent disinfecting effect against Acanthamoeba.

It was surprisingly found that the liquid formulation containing sodium dihydrogen phosphate, a basic isotonic agent and the predetermined poly(diallyldimethylammonium chloride) was applicable to various contact lenses.

Based on such findings, the present inventors have finally succeeded in inventing a liquid formulation containing sodium dihydrogen phosphate, a basic isotonic agent and the predetermined poly(diallyldimethylammonium chloride) as a solution to the problems of the present invention. As such, the present invention has been completed on the basis of the findings and successful examples that were first found or obtained by the present inventors.

According to the present invention, the following aspects of anti-Acanthamoeba liquid formulation are provided, respectively:

[1] An anti-Acanthamoeba liquid formulation, comprising sodium dihydrogen phosphate, a basic isotonic agent, and poly(diallyldimethylammonium chloride) with an average molecular weight of 10,000 to 1,100,000.

[2] The anti-Acanthamoeba liquid formulation according to item [1], wherein the ratio of the content of the basic isotonic agent relative to the content of sodium dihydrogen phosphate ([basic isotonic agent]/[sodium dihydrogen phosphate]) is 0.01 to 2, and the anti-Acanthamoeba liquid formulation has a pH value of 6.5 to 8.5.

[3] The anti-Acanthamoeba liquid formulation according to item [1] or [2], wherein the ratio of the content of poly(diallyldimethylammonium chloride) relative to the content of sodium dihydrogen phosphate ([poly(diallyldimethylammonium chloride)]/[sodium dihydrogen phosphate]) is 0.00005 to 0.01.

[4] The anti-Acanthamoeba liquid formulation according to any one of items [1] to [3], wherein the ratio of the content of poly(diallyldimethylammonium chloride) relative to the total content of sodium dihydrogen phosphate and the basic isotonic agent ([poly(diallyldimethylammonium chloride)]/([sodium dihydrogen phosphate]+[basic isotonic agent])) is 0.000001 to 1.

[5] The anti-Acanthamoeba liquid formulation according to any one of items [1] to [4], wherein the basic isotonic agent is at least one selected from the group consisting of 2-amino-2-hydroxymethyl-1,3-propanediol, 2-amino-2-ethyl-1,3-propanediol, and arginine, and a salt thereof.

[6] The anti-Acanthamoeba liquid formulation according to any one of items [1] to [5], wherein poly(diallyldimethylammonium chloride) is poly(diallyldimethylammonium chloride) with an average molecular weight of 100,000 to 1,000,000.

[7] The anti-Acanthamoeba liquid formulation according to any one of items [1] to [6], further comprising other isotonic

agent than the basic isotonic agent.

[8] The anti-Acanthamoeba liquid formulation according to item [7], wherein the other isotonic agent is at least one selected from the group consisting of sodium chloride, potassium chloride, 1,2-propanediol, 1,3-propanediol, mannitol, and sorbitol.

[9] The anti-Acanthamoeba liquid formulation according to any one of items [1] to [8], further comprising at least one quaternary ammonium salt selected from the group consisting of polyhexanide hydrochloride, alexidine, myristamidopropyl dimethylamine, benzalkonium chloride, benzethonium chloride, and polydronium chloride.

Effects of the Invention

According to the present invention, Acanthamoeba can be killed by a combination of components that have been proven to be safe in the field of ophthalmic liquid formulations. According to the present invention, various contact lenses can also be sterilized against Acanthamoeba without causing deformation or other problems. Therefore, the anti-Acanthamoeba liquid formulation according to one embodiment of the present invention can exhibit an excellent disinfecting effect against Acanthamoeba while being highly safe for ocular tissue and versatile enough to be applicable to a wide range of contact lenses.

The anti-Acanthamoeba liquid formulation according to one embodiment of the present invention can be employed for cleaning, rinsing, disinfecting and preserving of contact lenses, so that the formulation can be routinely utilized as MPS. According to the present invention, contact lens wearers can reduce or avoid the risk of contracting Acanthamoeba corneal infection caused by Acanthamoeba.

Description of Embodiments

While each embodiment of the present invention will now be described in detail, the present invention may take various forms to the extent that its objective can be achieved.

Unless otherwise specified, each term used herein is used in the meaning commonly used by those skilled in the art, in particular in the chemical fields relating to ocular lens such as contact lens, and should not be construed to have any meaning that is unduly limiting. Also, any speculations and theories herein are made on the basis of the knowledge and experiences of the present inventors and as such, the present invention is not bound by any such speculations and theories.

The term "content" is synonymous with concentration and amount used (amount added), and means the ratio of the amount of a component relative to the total amount of the final product. However, the total content of the components may not exceed 100%. Unless otherwise specified, the unit of content used herein means "mass/volume percent (w/v%)". If a commercial product is employed, the content of a component is preferably the amount of the component contained in the commercial product, but the content may also be the amount of the commercial product itself.

The term "and/or" as used herein means either any one of, any combination of two or more of, or combination of all of listed related items.

The terms "include," "comprise," and "contain" mean that an element(s) other than an element(s) as explicitly indicated can be added as inclusions, which are, for example, synonymous with "at least include," but encompasses the meaning of "consist of" and "substantially consist of". In other words, the terms may mean, for example, to include an element(s) as explicitly indicated as well as any one element or any two or more elements, to consist of an element(s) as explicitly indicated, or substantially consist of an element(s) as explicitly indicated. Such elements include limitations such as components, steps, conditions, and parameters.

The wording "to" for indicating a range of values is a range that include values preceding and following the wording, and also includes a range excluding one of the lower and upper limits included in the range. For example, "0 w/v% to 100 w/v%" means a range of values more than or equal to 0w/v%, of values less than or equal to 100 w/v%, and of values between 0 w/v% and 100 w/v%.

The number of digits of an integer equals to its significant figure. For example, 1 has one significant figure and 10 has two significant figures. For a decimal number, the number of digits after a decimal point equals to its significant figure. For example, 0.1 has one significant figure and 0.10 has two significant figures.

[Activity provided by anti-Acanthamoeba liquid formulation]

The anti-Acanthamoeba liquid formulation according to one embodiment of the present invention contains a combination of sodium dihydrogen phosphate, a basic isotonic agent, and poly(diallyldimethylammonium chloride) with a predetermined average molecular weight, thereby having the activity of killing Acanthamoeba, removing Acanthamoeba, and/or inhibiting the growth of Acanthamoeba (anti-Acanthamoeba activity). As used herein, causing the killing, removing or growth inhibiting of microorganisms may be referred to as the term "disinfection" or "sterilization".

The anti-Acanthamoeba liquid formulation according to one embodiment of the present invention has the anti-Acanthamoeba activity against microorganisms of the genus Acanthamoeba. The degree of the anti-Acanthamoeba activity may be confirmed using *Acanthamoeba castellanii* strain ATCC50370 as an indicator. The anti-Acanthamoeba activity of the anti-Acanthamoeba liquid formulation according to one embodiment of the present invention may be such that a disinfecting effect is observed against *Acanthamoeba castellanii* strain ATCC50370. For example, the activity may be such that, based on the method described in Examples below, when evaluated according to Evaluation Method 1 for disinfecting effect against Acanthamoeba, the dead amoeba rate is preferably 80% or more, more preferably 90% or more; and/or when evaluated according to Evaluation Method 2 for disinfecting effect against Acanthamoeba, the log reduction is preferably 0.5 or more, more preferably 1.0 or more, still more preferably 1.5 or more, and still even more preferably 2.0 or more.

The anti-Acanthamoeba liquid formulation according to one embodiment of the present invention has high safety for ocular tissue. The safety may be such that the formulation exhibits no remarkable adverse effect on ocular tissue. For example, the safety may be such that when evaluated based on the safety evaluation method described in Examples below, the relative colony formation rate is preferably 75% or more in adjusting the concentration to 2.5 v/v%, more preferably 75% or more in adjusting the concentration to 5 v/v%, still more preferably 75% or more in adjusting the concentration to 10 v/v% or more, and still even more preferably 75% or more in adjusting the concentration to 20 v/v%.

The anti-Acanthamoeba liquid formulation according to one embodiment of the present invention has contact lens applicability such that the formulation does not cause any change in power, basic curve, diameter or the like over a wide range of contact lenses. The contact lens applicability may be such that the formulation causes no remarkable change in the shape of contact lenses. For example, the contact lens applicability may be such that, when evaluated according to the contact lens applicability evaluation described in Examples below, the change in power is preferably within $\pm$0.25D, and/or the change in basic curve (BC) is preferably within $\pm$0.2mm, and/or the change in diameter (DIA) is preferably within $\pm$0.2mm.

[Active ingredient]

The anti-Acanthamoeba liquid formulation according to one embodiment of the present invention contains sodium dihydrogen phosphate, a basic isotonic agent, and poly(diallyldimethylammonium chloride) with a predetermined average molecular weight (hereinafter simply referred to as poly(diallyldimethylammonium chloride)) as active ingredients to exhibit the anti-Acanthamoeba activity.

Sodium dihydrogen phosphate is a type of inorganic phosphoric acid compound represented by the chemical formula $NaH_2PO_4$, and may be anhydride, monohydrate, dihydrate, or other hydrate, preferably sodium dihydrogen phosphate dihydrate that is widely used. In addition, the anti-Acanthamoeba liquid formulation according to one embodiment of the present invention may contain other inorganic phosphate compounds in addition to sodium dihydrogen phosphate. Such inorganic phosphate compounds include disodium hydrogen phosphate, trisodium phosphate, potassium dihydrogen phosphate, dipotassium hydrogen phosphate, tripotassium phosphate, calcium dihydrogen phosphate, and calcium hydrogen phosphate, and may be anhydride or hydrate. The other inorganic phosphate compounds may be used either individually or in combination of two or more of the above inorganic phosphate compounds.

So long as the content of sodium dihydrogen phosphate is an amount that exhibits the anti-Acanthamoeba activity when combined with the basic isotonic agent and poly(diallyldimethylammonium chloride), the content may be set as appropriate. Examples of the content include preferably 0.001 w/v% to 5 w/v%, more preferably 0.01 w/v% to 3 w/v%, still more preferably 0.05 w/v% to 2 w/v%, and still even more preferably 0.1 w/v% to 1 w/v%. While the total amount of the content of sodium dihydrogen phosphate and the content of the other inorganic phosphate compounds may be set as appropriate, examples of the total amount include preferably 0.01 w/v% to 5 w/v%, more preferably 0.05 w/v% to 3 w/v%, and still more preferably 0.1 w/v% to 2 w/v%.

The basic isotonic agent is an agent that has a basic group and is used to adjust the osmotic pressure of the anti-Acanthamoeba liquid formulation according to one embodiment of the present invention to a desired range (for example, to that of or near lacrimal fluid). Examples of the basic isotonic agent include ammonia, monoethanolamine, diethanolamine, triethanolamine, 2-amino-2-methyl-1 propanol, 2-amino-2-methyl-1,3-propanediol, 2-amino-2-hydroxymethyl-1,3-propanediol, 2-amino-2-ethyl-1,3-propanediol, and arginine, and a salt thereof, preferably organic buffering agents with amino group, and more preferably 2-amino-2-hydroxymethyl-1,3-propanediol, 2-amino-2-ethyl-1,3-propanediol, and arginine, from the viewpoint of little effect on ocular tissue and exhibiting the buffering action at a pH value near neutral. The basic isotonic agent may be used either individually or in combination of two or more of the above. The basic isotonic agent is preferably 2-amino-2-hydroxymethyl-1,3-propanediol, or a combination of 2-amino-2-hydroxymethyl-1,3-propanediol and other basic isotonic agents because of exhibiting a strong buffering action at a pH value near neutral.

So long as the content of the basic isotonic agent is an amount that exhibits the anti-Acanthamoeba activity when combined with sodium dihydrogen phosphate and poly(diallyldimethylammonium chloride), the content may be appropriately set in consideration of irritation to ocular tissue and osmotic pressure. Examples of the content include preferably

0.001 w/v% to 20 w/v%, more preferably 0.005 w/v% to 10 w/v%, and still more preferably 0.008 w/v% to 5 w/v%.

From the viewpoint of the anti-Acanthamoeba activity, the ratio between the content of sodium dihydrogen phosphate and the content of the basic isotonic agent is preferably within the predetermined range. Examples of the ratio of the content of the basic isotonic agent relative to the content of sodium dihydrogen phosphate ([basic isotonic agent]/[sodium dihydrogen phosphate]) include preferably 0.001 to 10, more preferably 0.005 to 5, still more preferably 0.01 to 3, and still even more preferably 0.04 to 2. The total content of sodium dihydrogen phosphate and the basic isotonic agent may be appropriately set in consideration of irritation to ocular tissue and osmotic pressure of the anti-Acanthamoeba liquid formulation according to one embodiment of the present invention.

Poly(diallyldimethylammonium chloride) is a cationic polymer having a predetermined average molecular weight and having a diallyldimethylammonium chloride unit represented by the following general formula (I) in its molecule. Poly(diallyldimethylammonium chloride) is also referred to as polyquaternium-6.

[Chemical formula 1]

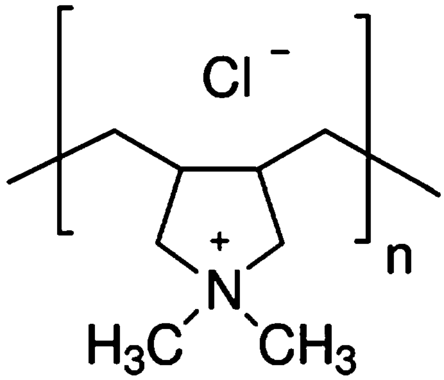

(I)

[In the formula, n represents an integer that is set so that the average molecular weight of the polymer falls within the range indicated above.]

The molecular weight of poly(diallyldimethylammonium chloride) is a predetermined average molecular weight, specifically from 10,000 to 1,100,000. In view of exhibiting the anti-Acanthamoeba activity while ensuring safety for ocular tissue, examples of the range of the average molecular weight of poly(diallyldimethylammonium chloride) include preferably 100,000 to 1,000,000, more preferably 120,000 to 800,000, and still more preferably 150,000 to 500,000. The average molecular weight represents the weight average molecular weight.

While the method of producing poly(diallyldimethylammonium chloride) is not particularly limited, examples of the method include a method subjecting the raw material aqueous solution monomer of diallyldimethylammonium chloride to cyclization polymerization using a radical polymerization initiator such as peroxide and light irradiation to obtain the linear homopolymer of poly(diallyldimethylammonium chloride).

Poly(diallyldimethylammonium chloride) used may be commercially available products. Examples of the commercially available product include "MERQUAT 100" (Lubrizol, molecular weight: 150,000), "ME Polymer H-40W" (Toho Chemical Industry, molecular weight: 240,000), "Cosmuat VG" (Senka, molecular weight: 200,000), "Unisense FPA1002L" (Senka, molecular weight: 500,000), "Unisense FPA7000E" (Senka, molecular weight: 1,200,000), "Flocare C106MV" (SNF, molecular weight: 150,000), and "Genamin PDAC" (Clariant International, molecular weight: 230,000) (the names of the polymers mentioned above represent registered trademarks or product names, respectively. The same applies to the polymers mentioned below). Furthermore, poly(diallyldimethylammonium chloride) used may be the Japanese Standards of Quasi-Drug Ingredients 2021 (JSQI) poly N,N-Dimethyl-3,4-dimethylene-pyrrolidinium chloride liquid.

So long as the content of poly(diallyldimethylammonium chloride) is an amount that exhibits the anti-Acanthamoeba activity when combined with sodium dihydrogen phosphate and the basic isotonic agent, the content may be appropriately set. In view of the fact that poly(diallyldimethylammonium chloride) significantly affects a disinfecting effect, examples of the content include preferably 0.00001 w/v% to 10 w/v%, more preferably 0.00005 w/v% to 1 w/v%, and still more preferably 0.00008 w/v% to 0.05 w/v%.

In order for a combination of sodium dihydrogen phosphate, the basic isotonic agent, and poly(diallyldimethylammonium chloride) to function effectively as active ingredients, the total amount thereof is preferably within a predetermined range. Examples of the total amount include preferably 0.01 w/v% to 10 w/v%, more preferably 0.05

w/v% to 5 w/v%, and still more preferably 0.1 w/v% to 3 w/v%.

From the viewpoint of the anti-Acanthamoeba activity, the ratio of the content of poly(diallyldimethylammonium chloride) relative to the content of sodium dihydrogen phosphate or the total amount of the content of sodium dihydrogen phosphate and the content of the basic isotonic agent is preferably within the predetermined range.

Examples of the ratio of the content of poly(diallyldimethylammonium chloride) relative to the content of sodium dihydrogen phosphate ([poly(diallyldimethylammonium chloride)]/[sodium dihydrogen phosphate]) include preferably 0.00001 to 1, more preferably 0.00005 to 0.5, and still more preferably 0.0001 to 0.1.

Examples of the ratio of the content of poly(diallyldimethylammonium chloride) relative to the total amount of the content of sodium dihydrogen phosphate and the content of the basic isotonic agent ([poly(diallyldimethylammonium chloride)]/([sodium dihydrogen phosphate]+[the basic isotonic agent]) include preferably 0.000001 to 1, more preferably 0.00001 to 0.5, and even more preferably 0.00003 to 0.1.

Given that the ratio of the content of poly(diallyldimethylammonium chloride) relative to the content of sodium dihydrogen phosphate or the total amount of the content of sodium dihydrogen phosphate and the content of the basic isotonic agent falls within the above-mentioned range, the anti-Acanthamoeba liquid formulation according to one embodiment of the present invention may have a disinfecting effect against Acanthamoeba while being safe for ocular tissue.

[Other components]

The anti-Acanthamoeba liquid formulation according to one embodiment of the present invention contains a combination of sodium dihydrogen phosphate, the basic isotonic agent and poly(diallyldimethylammonium chloride), and may further contain other components in addition to them. Examples of the other components include other isotonic agent than the basic isotonic agent. As used herein, the term "isotonic agent" simply mentioned represents an isotonic agent other than the basic isotonic agent, i.e., an isotonic agent that has no basic group.

While the isotonic agent is one having no basic group, examples of the isotonic agent include sodium chloride, potassium chloride, propylene glycol (PG), 1,2-propanediol, 1,3-propanediol, mannitol, sorbitol, sodium carbonate, sodium hydrogen carbonate, and glycerin. The isotonic agent may be used either individually or in combination of two or more of the above.

The content of the isotonic agent may be appropriately set so long as the content is an amount that can impart osmotic pressure suitable for ocular tissue to the anti-Acanthamoeba liquid formulation according to one embodiment of the present invention. Examples of the content include preferably 0 w/v% to 10 w/v%, more preferably 0.001 w/v% to 5 w/v%, still more preferably 0.01 w/v% to 3 w/v%, and still even more preferably 0.05 w/v% to 2 w/v% or 0.1 w/v% to 1.8 w/v%.

The other components are not particularly limited so long as they do not interfere with solving the problems of the present invention. Examples of the other components include additives that are usually contained in contact lens care solutions. Examples of such additives include disinfectants, surfactants, chelating agents, thickening agents, wetting agents, isotonic agents, and enzymes such as proteolytic or lipolytic enzymes. Some of the other components are listed below, but they are listed only as examples, and the other components are not limited thereto.

Examples of the disinfectant include biguanide-based disinfectants such as polyhexamethylene biguanide (PHMB), polyaminopropyl biguanide, alexidine and chlorhexidine gluconate (GCH); quaternary ammonium disinfectants such as myristamidopropyl dimethylamine, benzalkonium chloride, benzethonium chloride and polyduronium chloride; iodine-based disinfectants such as chlorhexidine gluconate and povidone iodine; and hydrogen peroxide disinfectants, preferably organic nitrogen-based disinfectants, and more preferably polyhexamethylene biguanide (PHMB). While the content of the disinfectant may be appropriately set, examples of the content include preferably 0 w/t% to 1.0 w/t% in view of ocular tissue irritation and safety.

Preferred examples of the surfactant include nonionic surfactants in view of impact on contact lenses. Examples of the non-ionic surfactant include polyoxyethylene polyoxypropylene glycol, ethylenediamine tetrapolyoxypropylene condensation products, polyoxyethylene alkyl ethers, polyoxyethylene alkyl phenyl ethers, polyoxyethylene hydrogenated castor oil, polyoxyalkyl esters, and polyoxyethylene sorbitan alkyl esters, preferably polyoxyethylene polyoxypropylene glycol, ethylenediamine tetrapolyoxypropylene condensation products, and polyoxyethylene hydrogenated castor oil.

Examples of polyoxyethylene polyoxypropylene glycol include polyoxyethylene (50) polyoxypropylene (40) glycol, polyoxyethylene (300) polyoxypropylene (55) glycol, polyoxyethylene (200) polyoxypropylene (40) glycol, polyoxyethylene (160) polyoxypropylene (30) glycol, polyoxyethylene (196) polyoxypropylene (67) glycol, and polyoxyethylene (200) polyoxypropylene (70) glycol. Some of them are commercially available as "Kolliphor P188", "Kolliphor P407" (BASF), "Pluronic (registered trademark) F-68, F-88, F-108, F-77, F-127, L-64, P-84, P-85" (ADEKA).

Examples of ethylenediamine tetrapolyoxypropylene condensation products include tetrafunctional block copolymer of polyoxyethylene polyoxypropylene copolymer and ethylenediamine. Some of them are commercially

available as "Tetoronic 904, 908, 1104, 1107, 1304, 1504" (BASF), "Synperonic T304, 707, 908" (CRODA), "Pluronic (registered trademark) TR-704, TR-913R" (ADEKA).

Examples of polyoxyethylene hydrogenated castor oil include polyoxyethylene hydrogenated castor oil 40, and polyoxyethylene hydrogenated castor oil 60. Some of them are commercially available as "Kolliphor RH-40" (BASF), "HCO-40, HCO-50, HCO-60" (Nikko Chemical).

The surfactant may be used either individually or in combination of two or more of the above. While the content of the surfactant may be appropriately set, examples of the content include preferably 0 w/t% to 5 w/t%, more preferably 0 w/t% to 2.5 w/t%, still more preferably 0 w/t% to 1 w/t%, and still even more preferably 0 w/t% to 0.5 w/t%, in view of ocular tissue safety and contact lens applicability.

While the chelating agent may be one normally used for ophthalmic liquid formulations, example of the chelating agent include polyvalent carboxylic acid such as edetic acid, sodium edetate, disodium edetate, ethylenediaminetetraacetic acid, citric acid and tartaric acid, and salts thereof. The chelating agent may be used either individually or in combination of two or more of the above. The content of the chelating agent may be appropriately set so long as the content is an amount normally used for ophthalmic liquid formulations. Examples of the content include preferably 0 w/t% to 10 w/t%, more preferably 0.001 w/t% to 5 w/t%, and still more preferably 0.01 w/t% to 1 w/t%, in view of ocular tissue safety.

While the thickening agent may be one normally used for ophthalmic liquid formulations, examples of the thickening agent include cellulose-based compounds such as hydroxyethylcellulose, hydroxypropylmethylcellulose (e.g., TC-5R), and sodium carboxycellulose; and vinyl-based compounds such as polyvinyl alcohol and carboxy vinyl polymer. While the content of the thickening agent may be appropriately set so long as the content is an amount normally used for ophthalmic liquid formulations. Examples of the content include preferably 0 w/t% to 5 w/t%, more preferably 0.001 w/t% to 2 w/t%, and still more preferably 0.01 w/t% to 1 w/t%, in view of ocular tissue safety.

While the wetting agent may be one normally used for ophthalmic liquid formulations, examples of the wetting agent include hyaluronic acid and a salt thereof, amino acid and a salt thereof, polyvinyl alcohol, and 2-methacryloyloxyethyl phosphorylcholine polymer.

The anti-Acanthamoeba liquid formulation according to one embodiment of the present invention is prepared by dissolving the components in a solvent such as water. The solvent may be preferably water normally used for ophthalmic liquid formulations such as purified water and ion-exchanged water. Examples of the content of water include preferably 90 wt% to 99.9 wt%, and more preferably 95 wt% to 99.5 wt%.

[Anti-Acanthamoeba liquid formulation]

The anti-Acanthamoeba liquid formulation according to one embodiment of the present invention may have various properties so long as the properties do not interfere with solving the problems of the present invention. For example, when the anti-Acanthamoeba liquid formulation according to one embodiment of the present invention is used to bring into contact with ocular tissue and contact lenses, the formulation preferably has properties that do not adversely affect ocular tissue and contact lenses.

In such a case, in order to mitigate ocular tissue irritation and to allow the basic isotonic agent to function as an isotonic agent, the anti-Acanthamoeba liquid formulation according to one embodiment of the present invention has, for example, preferably a pH value of 5.5 to 9.5, more preferably 6.0 to 9.0, and still more preferably 6.5 to 8.5; and preferably an osmotic pressure of 200 mOsm/L to 400 mOsm/L, and more preferably 220 mOsm/L to 350 mOsm/L.

The anti-Acanthamoeba liquid formulation according to one embodiment of the present invention may be produced according to methods of producing liquid formulations used in the ophthalmic field as known to those skilled in the art. For example, the formulation may be produced by adding sodium dihydrogen phosphate, the basic isotonic agent, and poly(diallyldimethylammonium chloride), and optionally other components to purified water, simultaneously or in sequence, and mixing them. The anti-Acanthamoeba liquid formulation according to one embodiment of the present invention may be subjected to filtration treatment to remove insoluble components, and to sterilization treatment to prevent contamination of microorganisms. The anti-Acanthamoeba liquid formulation according to one embodiment of the present invention is preferably a filtered liquid formulation, and/or preferably a sterilized liquid formulation. Specific examples of the method of producing the anti-Acanthamoeba liquid formulation according to one embodiment of the present invention may be found in the method described in Examples below.

The anti-Acanthamoeba liquid formulation according to one embodiment of the present invention may be used as a pharmaceutical or quasi-drug formulation that is a liquid composition. Examples of the pharmaceutical or quasi-drug formulation include eye drops, artificial tears, eye washes, and preservatives, cleaning agents and disinfectants for contact lenses.

The anti-Acanthamoeba liquid formulation according to one embodiment of the present invention is preferably a contact lens care liquid formulation because the formulation has safety for ocular tissue and applicability to a wide range of contact lenses. When the anti-Acanthamoeba liquid formulation according to one embodiment of the present invention is a contact lens care liquid formulation, the formulation may be used to clean, rinse, and disinfect contact lenses, and to store a

contact lens by placing the formulation together with a contact lens in a storage container.

The present invention will now be described in further detail with reference to Examples, which are not intended to limit the present invention. The present invention may take various embodiments to the extent that the objectives of the present invention are achieved.

## Examples

### [1. Preparation of test liquid formulation]

The test liquid formulations of Examples and Comparative Examples were prepared according to the compositions shown in Tables 1A to 1C. The unit for the component amount in Tables is "w/v%".

Specifically, each component listed in Tables 1A to 1C was weighed to reach the amount listed in Tables 1A to 1C. Next, the components were added to and dissolved in purified water to prepare a solution. The solution was then diluted to a total volume of 100 mL, and stirred sufficiently to become homogeneous. The obtained solution was filtered and sterilized using a PES membrane filter with a pore size of 0.2 μm to obtain a test liquid formulation.

### [2. Evaluation method 1 for disinfecting effect against Acanthamoeba]

Acanthamoeba (*Acanthamoeba castellanii* (ATCC50370)) was used as a test strain.

Acanthamoeba cells were cultured at 28 °C in a liquid medium, and the Acanthamoeba culture was then centrifuged. To the precipitated Acanthamoeba cells, one-fourth-strength Ringer's solution was added, and the cells were resuspended. The suspension was adjusted to an Acanthamoeba concentration of $1\times10^7$ cells/mL to $1\times10^8$ cells/mL to obtain an Acanthamoeba suspension. To 10 mL of each test liquid formulation of Examples and Comparative Examples, 0.1 mL of the Acanthamoeba suspension was inoculated. The inoculated test liquid formulation was subjected to disinfection treatment by incubation at 25 °C for 4 hours.

The test liquid formulation after the disinfection treatment was poured into 0.4 w/v% Trypan Blue Solution. The numbers of total cells, live cells, and dead cells in the poured test liquid formulation were determined under an optical microscope using a hemocytometer. Based on the numbers determined, the dead cell rate was calculated from the ratio of the number of dead cells relative to the number of total cells. When the calculated dead cell rate was equal to or more than 80%, the test liquid formulation used was evaluated as having a disinfecting effect.

### [3. Evaluation method 2 for disinfecting effect against Acanthamoeba]

Except that the Acanthamoeba concentration was set to be $5\times10^6$ cells/mL, the Acanthamoeba suspension was prepared and subjected to disinfection treatment in the same manner as [2. Evaluation Method 1 for disinfecting effect against Acanthamoeba] above.

To 1 mL of the test liquid formulation after the disinfection treatment, 9 mL of an inactivating agent (one-fourth-strength Ringer's solution containing 3.5% lecithin and 1.5% polysorbate) was added, and then a 10-fold serial dilution was prepared sequentially with one-fourth-strength Ringer's solution to obtain $10^{-1}$ to$10^{-6}$ diluted solutions.

To a 12-well culture plate, 2 mL of 1.5% agar solution was added, and 0.1 mL of *Escherichia coli* (ATCC8739) suspension (approximately $1\times10^{10}$ cells/mL) was seeded on the agar to prepare a plate for culture. Into four wells of the plate for culture, 1 mL each of the resulting diluted solutions ($10^{-1}$ to$10^{-6}$) was dispensed. The dispensed plate for culture was incubated at 28 °C for 14 days.

If Acanthamoeba cells remained alive during the culture, Acanthamoeba cells would propagate by feeding on E. coli cells. On the other hand, if Acanthamoeba cells were killed during the culture, E. coli cells would propagate. The control was prepared by adding the Acanthamoeba suspension to one-fourth-strength Ringer's solution. The numbers of positive and negative wells in the dilution series were counted according to the Reed and Muench method. The $LD_{50}$ value was calculated based on the total number of wells to determine the Logarithmic Reduction (Log Reduction) for each test liquid formulation. The test liquid formulation showing a Log Reduction equal to or more than 0.5 was evaluated as having a disinfecting effect.

### [4. Evaluation method for disinfecting effect against fungi]

*Candida albicans* (ATCC10231) was used as a test strain to evaluate a disinfecting effect against fungi.

Candida cells were cultured on an agar slant medium at 35 °C for 18 hours. The cultured Candida cells were suspended in Dulbecco's phosphate-buffered saline. The suspension was collected and used as a fungal stock solution. The fungal stock solution was diluted with Dulbecco's phosphate-buffered saline to adjust the concentration to $10^5$ to $10^6$ CFU/mL to prepare a fungal suspension.

To 10 mL of each test liquid formulation of Examples and Comparative Examples, 0.1 mL of the fungal suspension was added and inoculated. The inoculated test liquid formulation was subjected to disinfection treatment by incubation at 25 °C for 4 hours and 6 hours.

To 9 mL of Neutralizing Broth, 1 mL of the test liquid formulation after the disinfection treatment was added. The mixture was then used to prepare a 10-fold serial dilution in Dulbecco's phosphate-buffered saline to obtain diluted test liquid formulations subjected to the disinfection treatment. Into a disposable dish, 1 mL each of three optionally selected diluted test liquid formulations was dispensed, and Sabouraud dextrose agar medium was poured into the dish, and mixed. The resulting dish was incubated at 35 °C for 48 hours, and the number of viable fungi was determined from the number of colonies formed.

The initial number of fungi, corresponding to the number of fungi in the fungal suspension, was determined by the agar plate method using a 10-fold serial dilution sequentially prepared in Dulbecco's phosphate-buffered saline as described above.

Based on the initial number of fungi determined and the number of viable fungi obtained using the diluted test liquid formulation after the disinfection treatment, the Log Reduction after the disinfection treatment in each test liquid formulation was calculated according to the following formula:

$$\text{Log Reduction} = \text{Initial number of fungi} - \text{Number of viable fungi in test liquid formulation}$$

The disinfecting effect was evaluated according to the primary criteria of the ISO stand-alone test. The test liquid formulation showing a Log Reduction equal to or more than 1.0 during the predetermined disinfection time was evaluated as having a disinfecting effect (Reference Document: ISO 14729).

[5. Safety evaluation method]

V79 cells, derived from Chinese hamster lung-derived cells, were pre-cultured in a $CO_2$ incubator set at 37 °C, and then trypsinized and collected. The collected cells were suspended in Eagle's minimal essential medium containing fetal bovine serum, which is a cell culture medium, to obtain a cell suspension.

Each test liquid formulation of Examples and Comparative Examples was dispensed into each well of a culture plate so that the concentration of the liquid formulation was 20%, 10%, 5%, or 2.5% (v/v%). The cell suspension was then added to each well so that the number of cells was 100. In addition, Eagle's minimum essential medium containing fetal bovine serum was added to each well to bring the total volume to 2,000 μL. For example, when the concentration of the liquid formulation was 20%, 1,500 μL of the medium, 100 μL of the cell suspension (1,000 cells/mL) and 400 μL of the test liquid formulation were poured into the well, and mixed. The plate was incubated in a $CO_2$ incubator set at 37 °C for 7 days. The control was prepared by the culture using the cell culture medium instead of the test liquid formulation. The relative colony formation rate at each concentration point of each test liquid formulation was calculated according to the following formula.

Relative colony formation rate (%) = (number of colonies at a concentration point/number of colonies of the control) × 100

The relative colony formation rate corresponded to the safety for ocular tissue, and the safety of each test liquid formulation was evaluated by comparison with the relative colony formation rate at the same concentration as follows:

+: Relative colony formation rate equal to or more than 75%
±: Relative colony formation rate more than 50% and less than 75%
-: Relative colony formation rate equal to or less than 50%

[6. Contact lens applicability evaluation]

As the test lenses, "SEED 2weekFineUV Plus (Group 1)," "SEED 2weekPure (Group 4)," "J&J ACUVUE OASYS (Group VC)," "Alcon AIR OPTIX HydraGlyde (Group VC)," and "CooperVision Biofinity (Group VC)" were used.

Each test lens was taken out of the blister pack, and surface moisture on the lens was removed. The test lens after removing surface moisture was immersed in Dulbecco's phosphate-buffered saline, and parameters before treatment with the test liquid formulation (Power, Base Curve (BC) and Diameter (DIA)) were measured.

The test lens was then washed by rubbing the lens with fingers 30 times (cycle treatment) using each test liquid formulation of Examples and Comparative Examples. The test lens after the cycle treatment was immersed in the

phosphate buffer, and the above parameters were measured.

The contact lens applicability of each test liquid formulation was evaluated by the amount of change before and after the cycle treatment, according to the contact lens approval criteria as follow:

+: The amount of change in Power was within ±0.25 D, that in BC was within ± 0.2 mm, and that in DIA was within ± 0.2 mm.
-: The amount of change in Power was more than ±0.25 D, that in BC was more than ±0.2 mm, or that in DIA was more than ±0.2 mm.

[7. Evaluation method for property of test liquid formulation]

For each test liquid formulation of Examples and Comparative Examples, the pH value was measured at room temperature using the pH meter "Main Unit: Seven Compact S220, Electrode: InLab MicroPro-ISM" (Mettler Toledo); the osmotic pressure was measured using the osmometer "AD-3250" (Advanced Instruments).

[8. Evaluation results]

The measurement results of the pH value and osmotic pressure (mOsm) of each test liquid formulation are shown in Tables 1A to 1C. The pH values of the test liquid formulations of Examples 1 to 22 were 6.55 to 8.52, and the osmotic pressures of the formulations were 230 to 339. Therefore, the test liquid formulations were found to be applicable as a liquid formulation for contact lenses.

The test liquid formulations of Examples 1 to 11 and Comparative Examples 1 to 6 were evaluated for the disinfecting effect against Acanthamoeba according to Evaluation Method 1 for disinfecting effect against Acanthamoeba. The results are shown in Table 1A and Table 1C. As shown in the tables, all of the test liquid formulations of Examples 1 to 11, which contained a combination of sodium dihydrogen phosphate dihydrate, a basic isotonic agent, and poly(diallyldimethylammonium chloride) having an average molecular weight of 10,000 to 1,100,000, were found to have a dead amoeba rate of 80% or more, indicating a sufficient disinfecting effect against Acanthamoeba. In contrast, all of the test liquid formulations of Comparative Examples 1 to 6, which contained sodium dihydrogen phosphate dihydrate and a basic isotonic agent but contained poly(diallyldimethylammonium chloride) having an average molecular weight of 8,500 or 1,200,000, or did not contain any poly(diallyldimethylammonium chloride), were found to have a dead amoeba rate of 70% or less, indicating no disinfecting effect against Acanthamoeba.

In addition, the test liquid formulations of Examples 1 to 11 that were found to have a disinfecting effect against Acanthamoeba had a ratio of the content of the basic isotonic agent relative to the content of sodium dihydrogen phosphate ([basic isotonic agent]/[sodium dihydrogen phosphate]) in the range between 0.01 and 2, whereas the test liquid formulations of Comparative Examples 3 to 6 that were found to have no disinfecting effect against Acanthamoeba had a ratio outside the above range.

The test liquid formulations of Examples 12 to 22 and Comparative Examples 7 to 8 were evaluated for the disinfecting effect against Acanthamoeba according to Evaluation Method 2 for disinfecting effect against Acanthamoeba. The results are shown in Table 1B and Table 1C. As shown in the tables, all of the test liquid formulations of Examples 12 to 22, which contained a combination of sodium dihydrogen phosphate dihydrate, a basic isotonic agent, and poly(diallyldimethylammonium chloride) having an average molecular weight of 10,000 to 1,100,000, were found to have a Log Reduction of 2.00 to 2.33, i.e., more than 0.5, indicating a sufficient disinfecting effect against Acanthamoeba. In contrast, all of the test liquid formulations of Comparative Examples 7 to 8, which contained sodium dihydrogen phosphate dihydrate and a basic isotonic agent but contained poly(diallyldimethylammonium chloride) having an average molecular weight of 8,500, or did not contain any poly(diallyldimethylammonium chloride), were found to have a Log Reduction of 0.25, indicating no disinfecting effect against Acanthamoeba.

Therefore, according to the evaluation methods for disinfecting effect against Acanthamoeba executed, it was found that a liquid formulation containing a combination of sodium dihydrogen phosphate dihydrate, a basic isotonic agent, and poly(diallyldimethylammonium chloride) having an average molecular weight of 10,000 to 1,100,000 had an excellent disinfecting effect against Acanthamoeba.

The test liquid formulations of Examples 12 to 15 and Comparative Examples 7 to 8 were evaluated for the disinfecting effect against fungi according to Evaluation Method for disinfecting effect against fungi. The results are shown in Table 1B and Table 1C. As shown in the tables, the test liquid formulations of Examples were found not to be necessarily effective in disinfecting fungi, because some of the formulations had a log reduction more than 1.0 while others had a log reduction less than 1.0, even when the disinfection time was taken into account. In contrast, the test liquid formulation of Comparative Example 8, which contained poly(diallyldimethylammonium chloride) having an average molecular weight of 8,500, had no disinfecting effect against fungi, whereas the test liquid formulation of Comparative Example 7, which contained sodium dihydrogen phosphate dihydrate and a basic isotonic agent but did not contain poly(diallyldimethylammonium chloride), had a log reduction of more than 1.0 at any disinfection time to exhibit a disinfecting effect against fungi.

From the results above, it was surprisingly found that a liquid formulation containing poly(diallyldimethylammonium chloride) having an average molecular weight of 10,000 to 1,100,000 in combination with sodium dihydrogen phosphate dihydrate and a basic isotonic agent exhibited a specific disinfecting effect against Acanthamoeba while possibly having the reduced disinfecting effect against fungi as compared with a liquid formulation containing no poly(diallyldimethylammonium chloride).

For the safety and applicability to contact lenses, all of the test liquid formulations of Examples 12 to 22 were evaluated as "+" at all concentrations tested for safety and for all contact lenses tested for applicability. When the evaluation results of the safety and applicability to contact lenses were considered together with those of the pH value and osmotic pressure, a liquid formulation containing a combination of sodium dihydrogen phosphate dihydrate, a basic isotonic agent, and poly(diallyldimethylammonium chloride) having an average molecular weight of 10,000 to 1,100,000 was found to have an excellent disinfecting effect against Acanthamoeba while being a liquid formulation applicable to contact lenses.

[Table 1A]

| | | | Examples | | | | | | | | | | |
|---|---|---|---|---|---|---|---|---|---|---|---|---|---|
| | | | 1 | 2 | 3 | 4 | 5 | 6 | 7 | 8 | 9 | 10 | 11 |
| Components | Inorganic phosphoric acid compound | $Na_2HPO_4$ | - | 0.125 | - | - | - | - | - | - | - | - | - |
| | | $NaH_2PO_4 \cdot 2H_2O$ | 0.312 | 0.15 | 0.78 | 0.78 | 0.78 | 0.78 | 0.156 | 0.156 | 0.78 | 0.468 | 0.78 |
| | Basic isotonic agent | 2-Amino-2-hydroxymethyl-1,3-propanediol | 0.1 | 0.05 | - | - | 0.5 | 1.0 | 0.12 | 0.12 | 0.3 | 0.36 | 0.3 |
| | | 2-Amino-2-ethyl-1,3-propanediol | - | - | 0.65 | - | 0.5 | - | - | - | 0.3 | - | 0.1 |
| | | Arginine | - | - | - | 1.0 | - | - | - | - | - | - | 0.2 |
| | Poly(diallyldimethylammonium chloride) | PQ-6 (molecular weight of 8500) | - | - | - | - | - | - | - | - | - | - | - |
| | | PQ-6 (molecular weight of 150000) | 0.002 | - | - | - | 0.0001 | - | 0.01 | - | - | - | - |
| | | PQ-6 (molecular weight of 200000) | - | - | - | - | - | - | - | 0.01 | - | - | - |
| | | PQ-6 (molecular weight of 230000) | - | - | - | - | - | - | - | - | 0.01 | - | - |
| | | PQ-6 (molecular weight of 240000) | - | - | 0.001 | 0.001 | - | - | - | - | - | 0.01 | - |
| | | PQ-6 (molecular weight of 500000) | - | 0.002 | - | - | - | 0.0001 | - | - | - | - | 0.01 |
| | | PQ-6 (molecular weight of 1200000) | - | - | - | - | - | - | - | - | - | - | - |
| | Isotonic agent | NaCl | 0.35 | 0.45 | 0.35 | 0.28 | - | 0.28 | 0.63 | - | - | 0.43 | 0.17 |
| | | PG | 0.9 | - | 0.2 | | 0.25 | 0.5 | - | 1.5 | 0.42 | - | - |
| | | D-Mannitol | 0.4 | - | 0.2 | 0.4 | - | - | - | - | - | 0.4 | 1.2 |
| | | D-Sorbitol | - | 0.85 | - | - | - | - | - | - | - | - | - |
| | | Potassium chloride | 0.01 | - | - | - | - | 0.1 | - | - | - | - | - |
| | | 1,3-Propanediol | | - | - | - | 0.25 | - | - | - | 0.4 | - | - |
| | Chelating agent | EDTA・2Na | 0.02 | 0.02 | 0.02 | 0.02 | 0.02 | 0.02 | 0.05 | 0.05 | 0.02 | 0.02 | 0.02 |
| | | Sodium citrate・$2H_2O$ | - | - | - | - | - | - | - | - | - | 0.1 | - |
| | Surfactant | TR-704 | - | - | - | - | - | - | - | - | - | - | - |
| | | T908 | - | - | - | - | - | - | - | - | - | - | - |
| | Thickening agent | TC-5R | - | - | - | - | - | - | - | - | - | - | - |
| | pH adjusting agent | 1M NaOH | - | - | - | - | - | - | - | - | - | - | 0.75 |
| | Disinfectant | PHMB | - | - | - | - | - | - | - | - | - | - | - |
| | Water | Purified water | Residual | Residual | Residual | Residual | Residual | Residual | Residual | Residual | Residual | Residual | Residual |
| | Total | | 100 | 100 | 100 | 100 | 100 | 100 | 100 | 100 | 100 | 100 | 100 |
| | [Basic isotonic agent]/[$NaH_2PO_4 \cdot 2H_2O$] | | 0.32 | 0.33 | 0.83 | 1.28 | 1.28 | 1.28 | 0.77 | 0.77 | 0.77 | 0.77 | 0.77 |
| Evaluation results | pH | | 6.55 | 7.01 | 8.41 | 7.91 | 8.52 | 8.12 | 7.17 | 7.36 | 7.67 | 7.37 | 7.61 |
| | mOsm | | 309 | 235 | 283 | 233 | 235 | 339 | 230 | 236 | 245 | 245 | 246 |
| | Disinfecting effect against Acanthamoeba 1 (ATCC 50370) | Disinfection time 4hr (Dead amoeba rate;%) | 93.8 | 100 | 100 | 100 | 98.4 | 98.8 | 98.6 | 100 | 99.4 | 95.3 | 100 |
| | Disinfecting effect against Acanthamoeba 2 (ATCC 50370) | Disinfection time 4hr (Log reduction) | | | | | | | | | | | |
| | Disinfecting effect against fungi (ATCC 10231) | Disinfection time 4hr (Log reduction) | | | | | | | | | | | |
| | | Disinfection time 6hr (Log reduction) | | | | | | | | | | | |

[Table 1B]

| | | | Examples | | | | | | | | | | |
|---|---|---|---|---|---|---|---|---|---|---|---|---|---|
| | | | 12 | 13 | 14 | 15 | 16 | 17 | 18 | 19 | 20 | 21 | 22 |
| Components | Inorganic phosphoric acid compound | $Na_2HPO_4$ | 0.125 | - | - | - | - | - | - | - | - | - | 1.125 |
| | | $NaH_2PO_4 \cdot 2H_2O$ | 0.15 | 0.78 | 0.78 | 0.78 | 0.156 | 0.195 | 0.312 | 0.312 | 0.156 | 0.78 | 0.195 |
| | Basic isotonic agent | 2-Amino-2-hydroxymethyl-1,3-propanediol | 0.1 | 0.6 | 0.6 | 0.6 | 0.12 | 0.15 | 0.24 | 0.24 | 0.12 | 0.6 | 0.01 |
| | | 2-Amino-2-ethyl-1,3-propanediol | - | - | - | - | - | - | - | - | - | - | - |
| | | Arginine | - | - | - | - | - | - | - | - | - | - | - |
| | Poly(diallyldimethylammonium chloride) | PQ-6 (molecular weight of 8500) | - | - | - | - | - | - | - | - | - | - | - |
| | | PQ-6 (molecular weight of 150000) | - | - | - | - | - | - | - | - | - | - | - |
| | | PQ-6 (molecular weight of 200000) | - | - | - | - | - | - | - | - | - | - | - |
| | | PQ-6 (molecular weight of 230000) | - | - | - | - | - | - | - | - | - | - | - |
| | | PQ-6 (molecular weight of 240000) | 0.0004 | 0.0004 | 0.0004 | 0.0004 | 0.0004 | 0.0004 | 0.0004 | 0.0004 | 0.0004 | 0.0004 | 0.0004 |
| | | PQ-6 (molecular weight of 500000) | - | - | - | - | - | - | - | - | - | - | - |
| | | PQ-6 (molecular weight of 1200000) | - | - | - | - | - | - | - | - | - | - | - |
| | Isotonic agent | NaCl | 0.45 | 0.20 | - | 0.17 | 0.56 | 0.54 | 0.49 | 0.27 | 0.63 | 0.28 | - |
| | | PG | - | - | 0.8 | - | - | - | - | 0.5 | - | - | - |
| | | D-Mannitol | - | - | - | 1.2 | 0.4 | 0.4 | 0.4 | 0.4 | - | 0.4 | 0.4 |
| | | D-Sorbitol | 0.85 | 0.85 | - | - | - | - | - | - | - | - | - |
| | | Potassium chloride | - | - | - | - | - | - | - | - | - | - | - |
| | | 1,3-Propanediol | - | - | - | - | - | - | - | - | - | - | - |
| | Chelating agent | EDTA・2Na | 0.02 | 0.02 | 0.02 | 0.02 | 0.02 | 0.02 | 0.02 | 0.02 | 0.05 | 0.02 | 0.02 |
| | | Sodium citrate・$2H_2O$ | 0.1 | 0.1 | 0.1 | - | 0.1 | 0.1 | 0.1 | 0.1 | 0.1 | 0.1 | 0.1 |
| | Surfactant | TR-704 | 0.1 | 0.1 | 0.1 | - | - | - | - | - | - | - | - |
| | | T908 | - | - | - | 0.1 | 0.1 | 0.1 | 0.1 | 0.1 | - | 0.1 | - |
| | Thickening agent | TC-5R | 0.1 | 0.1 | 0.1 | 0.1 | 0.1 | 0.1 | 0.1 | 0.1 | - | 0.1 | - |
| | pH adjusting agent | 1M NaOH | - | - | - | - | - | - | - | - | - | 1.0 | - |
| | Disinfectant | PHMB | 0.0001 | 0.0001 | 0.0001 | 0.0001 | 0.0001 | 0.0001 | 0.0001 | 0.0001 | 0.0001 | 0.0001 | 0.0001 |
| | Water | Purified water | Residual | Residual | Residual | Residual | Residual | Residual | Residual | Residual | Residual | Residual | Residual |
| | Total | | 100 | 100 | 100 | 100 | 100 | 100 | 100 | 100 | 100 | 100 | 100 |
| | [Basic isotonic agent]/[$NaH_2PO_4 \cdot 2H_2O$] | | 0.67 | 0.77 | 0.77 | 0.77 | 0.77 | 0.77 | 0.77 | 0.77 | 0.77 | 0.77 | 0.05 |
| Evaluation results | pH | | 7.41 | 7.40 | 7.42 | 7.46 | 7.51 | 7.43 | 7.47 | 7.50 | 7.46 | 7.85 | 7.57 |
| | mOsm | | 248 | 243 | 240 | 240 | 238 | 239 | 239 | 238 | 239 | 250 | 241 |
| | Disinfecting effect against Acanthamoeba 1 (ATCC 50370) | Disinfection time 4hr (Dead amoeba rate;%) | | | | | | | | | | | |
| | Disinfecting effect against Acanthamoeba 2 (ATCC 50370) | Disinfection time 4hr (Log reduction) | 2.33 | 2.33 | 2.33 | 2.33 | 2.33 | 2.33 | 2.33 | 2.33 | 2.33 | 2.00 | 2.00 |
| | Disinfecting effect against fungi (ATCC 10231) | Disinfection time 4hr (Log reduction) | 0.5 | 0.7 | 1.0 | 1.0 | - | 0.9 | 0.9 | 1.0 | 1.0 | 1.1 | 1.0 |
| | | Disinfection time 6hr (Log reduction) | 0.9 | 1.4 | 1.7 | 1.9 | - | 1.4 | 1.7 | 1.5 | 1.5 | 1.5 | 1.5 |

[Table 1C]

| | | | Comparative Examples | | | | | | | |
|---|---|---|---|---|---|---|---|---|---|---|
| | | | 1 | 2 | 3 | 4 | 5 | 6 | 7 | 8 |
| Components | Inorganic phosphoric acid compound | $Na_2HPO_4$ | - | - | - | - | 0.65 | 0.65 | 0.125 | - |
| | | $NaH_2PO_4 \cdot 2H_2O$ | 0.78 | 0.312 | 0.156 | 0.156 | 0.78 | 0.78 | 0.15 | 0.78 |
| | Basic isotonic agent | 2-Amino-2-hydroxymethyl-1,3-propanediol | 0.3 | 0.24 | 0.35 | 0.35 | 0.005 | 0.005 | 0.1 | 0.6 |
| | | 2-Amino-2-ethyl-1,3-propanediol | 0.3 | - | - | - | - | - | - | - |
| | | Arginine | - | - | - | - | - | - | - | - |
| | Poly(diallyldimethylammonium chloride) | PQ-6 (molecular weight of 8500) | 0.01 | - | - | - | - | - | - | 0.0005 |
| | | PQ-6 (molecular weight of 150000) | - | - | - | - | - | - | - | - |
| | | PQ-6 (molecular weight of 200000) | - | - | - | - | - | - | - | - |
| | | PQ-6 (molecular weight of 230000) | - | - | - | - | - | - | - | - |
| | | PQ-6 (molecular weight of 240000) | - | - | - | - | - | - | - | - |
| | | PQ-6 (molecular weight of 500000) | - | - | - | - | - | - | - | - |
| | | PQ-6 (molecular weight of 1200000) | - | - | 0.0001 | 0.001 | 0.0001 | 0.001 | - | - |
| | Isotonic agent | NaCl | - | 0.49 | 0.7 | 0.7 | 0.25 | 0.25 | 0.45 | 0.37 |
| | | PG | 0.2 | - | - | - | - | - | - | - |
| | | D-Mannitol | - | 0.4 | - | - | | | - | - |
| | | D-Sorbitol | 0.85 | - | - | - | - | - | 0.85 | - |
| | | Potassium chloride | - | - | - | - | - | - | - | - |
| | | 1,3-Propanediol | - | - | - | - | - | - | - | - |
| | Chelating agent | EDTA・2Na | 0.02 | 0.02 | 0.1 | 0.1 | 0.02 | 0.02 | 0.02 | 0.02 |
| | | Sodium citrate・$2H_2O$ | - | - | - | - | - | - | 0.1 | - |
| | Surfactant | TR-704 | - | - | - | - | - | - | 0.1 | - |
| | | T908 | - | - | - | - | - | - | - | 0.1 |
| | Thickening agent | TC-5R | - | - | - | - | - | - | 0.1 | 0.1 |
| | pH adjusting agent | 1M NaOH | - | - | - | - | 3.5 | 3.5 | - | - |
| | Disinfectant | PHMB | - | 0.001 | - | - | - | - | 0.0001 | - |
| | Water | Purified water | Residual | Residual | Residual | Residual | Residual | Residual | Residual | Residual |
| | Total | | 100 | 100 | 100 | 100 | 100 | 100 | 100 | 100 |
| | [Basic isotonic agent]/[$NaH_2PO_4 \cdot 2H_2O$] | | 0.77 | 0.77 | 2.24 | 2.24 | 0.01 | 0.01 | 0.67 | 0.77 |
| Evaluation results | pH | | 7.67 | 7.33 | 8.39 | 8.38 | 7.38 | 7.38 | 7.41 | - |
| | mOsm | | 206 | 230 | 273 | 273 | 299 | 298 | 248 | 236 |
| | Disinfecting effect against Acanthamoeba 1 (ATCC 50370) | Disinfection time 4hr (Dead amoeba rate;%) | 50.5 | 10.3 | 5.6 | 4.8 | 29.0 | 66.4 | | |
| | Disinfecting effect against Acanthamoeba 2 (ATCC 50370) | Disinfection time 4hr (Log reduction) | | | | | | | 0.25 | 0.25 |
| | Disinfecting effect against fungi (ATCC 10231) | Disinfection time 4hr (Log reduction) | | | | | | | 1.0 | 0.1 |
| | | Disinfection time 6hr (Log reduction) | | | | | | | 1.5 | 0.2 |

[Industrial applicability]

The anti-Acanthamoeba liquid formulation according to one embodiment of the present invention has high safety for ocular tissue, applicability to a wide range of contact lenses, and an excellent disinfecting effect against Acanthamoeba, so that the liquid formulation can be used as a contact lens care liquid formulation such as MPS to reduce or prevent the risk of contact lens wearers being affected by keratitis caused by Acanthamoeba, thereby contributing to the health and welfare of the wearers.

Cross-reference of related applications

The present application claims the benefit of priority to Japanese Patent Application No. 2023-123521, filed on July 28, 2023, the disclosure of which is incorporated herein by reference in its entirety. In addition, the disclosure of all the documents described herein including Patent Documents 1 to 5 is incorporated herein by reference in its entirety.

## Claims

1. An anti-Acanthamoeba liquid formulation, comprising sodium dihydrogen phosphate, a basic isotonic agent, and poly (diallyldimethylammonium chloride) with an average molecular weight of 10,000 to 1,100,000.

2. The anti-Acanthamoeba liquid formulation according to claim 1, wherein the ratio of the content of the basic isotonic agent relative to the content of sodium dihydrogen phosphate ([basic isotonic agent]/[sodium dihydrogen phosphate]) is 0.01 to 2, and the anti-Acanthamoeba liquid formulation has a pH value of 6.5 to 8.5.

3. The anti-Acanthamoeba liquid formulation according to claim 1, wherein the ratio of the content of poly(diallyldimethylammonium chloride) relative to the content of sodium dihydrogen phosphate ([poly(diallyldimethylammonium chloride)]/[sodium dihydrogen phosphate]) is 0.00005 to 0.01.

4. The anti-Acanthamoeba liquid formulation according to claim 1, wherein the ratio of the content of poly(diallyldimethylammonium chloride) relative to the total amount of the content of sodium dihydrogen phosphate and the content of the basic isotonic agent ([poly(diallyldimethylammonium chloride)]/([sodium dihydrogen phosphate] +[basic isotonic agent])) is 0.000001 to 1.

5. The anti-Acanthamoeba liquid formulation according to claim 1, wherein the basic isotonic agent is at least one selected from the group consisting of 2-amino-2-hydroxymethyl-1,3-propanediol, 2-amino-2-ethyl-1,3-propanediol, and arginine, and a salt thereof.

6. The anti-Acanthamoeba liquid formulation according to claim 1, wherein poly(diallyldimethylammonium chloride) is poly(diallyldimethylammonium chloride) with an average molecular weight of 100,000 to 1,000,000.

7. The anti-Acanthamoeba liquid formulation according to claim 1, further comprising other isotonic agent than the basic isotonic agent.

8. The anti-Acanthamoeba liquid formulation according to claim 7, wherein the other isotonic agent is at least one selected from the group consisting of sodium chloride, potassium chloride, 1,2-propanediol, 1,3-propanediol, mannitol, and sorbitol.

9. The anti-Acanthamoeba liquid formulation according to claim 1, further comprising at least one quaternary ammonium salt selected from the group consisting of polyhexanide hydrochloride, alexidine, myristamidopropyl dimethylamine, benzalkonium chloride, benzethonium chloride, and polydronium chloride.

## INTERNATIONAL SEARCH REPORT

International application No.
**PCT/JP2024/026550**

### A. CLASSIFICATION OF SUBJECT MATTER

***A61L 12/14***(2006.01)i; ***A61K 31/785***(2006.01)i; ***A61K 47/02***(2006.01)i; ***A61K 47/18***(2017.01)i; ***A61L 12/08***(2006.01)i; ***A61L 12/10***(2006.01)i; ***A61L 12/12***(2006.01)i; ***A61P 33/04***(2006.01)i; *A61L 101/16*(2006.01)n; *A61L 101/34*(2006.01)n; *A61L 101/46*(2006.01)n

FI: A61L12/14 106; A61L12/08; A61L12/10; A61L12/12; A61K31/785; A61K47/02; A61K47/18; A61P33/04; A61L101:46; A61L101:34; A61L101:16

According to International Patent Classification (IPC) or to both national classification and IPC

### B. FIELDS SEARCHED

Minimum documentation searched (classification system followed by classification symbols)

A61L12/14; A61K31/785; A61K47/02; A61K47/18; A61L12/08; A61L12/10; A61L12/12; A61P33/04; A61L101/16; A61L101/34; A61L101/46

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| A | JP 2002-143277 A (OPHTECS CORP.) 21 May 2002 (2002-05-21) abstract | 1-9 |
| A | JP 2013-234176 A (SANTEN PHARMACEUTICAL CO., LTD.) 21 November 2013 (2013-11-21) abstract | 1-9 |
| A | JP 2014-218461 A (SEED CO., LTD.) 20 November 2014 (2014-11-20) abstract | 1-9 |
| A | JP 2018-35151 A (ROHTO PHARMACEUT CO., LTD.) 08 March 2018 (2018-03-08) abstract | 1-9 |
| A | JP 2011-33701 A (SEED CO., LTD.) 17 February 2011 (2011-02-17) abstract | 1-9 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **04 October 2024** | **22 October 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**

International application No.
**PCT/JP2024/026550**

**C. DOCUMENTS CONSIDERED TO BE RELEVANT**

| Category* | Citation of document, with indication, where appropriate, of the relevant passages | Relevant to claim No. |
|---|---|---|
| A | JP 2006-23512 A (ROHTO PHARMACEUT CO., LTD.) 26 January 2006 (2006-01-26) abstract | 1-9 |
| A | JP 2004-339104 A (ROHTO PHARMACEUT CO., LTD.) 02 December 2004 (2004-12-02) abstract | 1-9 |

Form PCT/ISA/210 (second sheet) (July 2022)

**INTERNATIONAL SEARCH REPORT**
**Information on patent family members**

International application No.
**PCT/JP2024/026550**

| Patent document cited in search report | Publication date (day/month/year) | Patent family member(s) | Publication date (day/month/year) |
|---|---|---|---|
| JP 2002-143277 A | 21 May 2002 | (Family: none) | |
| JP 2013-234176 A | 21 November 2013 | WO 2013/154209 A1 abstract | |
| JP 2014-218461 A | 20 November 2014 | (Family: none) | |
| JP 2018-35151 A | 08 March 2018 | (Family: none) | |
| JP 2011-33701 A | 17 February 2011 | (Family: none) | |
| JP 2006-23512 A | 26 January 2006 | (Family: none) | |
| JP 2004-339104 A | 02 December 2004 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2002143277 A
- JP 2011246458 A
- JP 2013234176 A
- JP 2014218461 A
- JP 2018035151 A
- JP 2023123521 A